# EUROPÄISCHE PATENTSCHRIFT

(11) **EP 0 556 684 B1**
(45) Veröffentlichungstag und Bekanntmachung des Hinweises auf die Patenterteilung: **22.05.1996**
(21) Anmeldenummer: 93101949.1
(22) Anmeldetag: 08.02.1993
(51) Int. Cl.: C07D 213/61, C07D 213/38

(54) **Verfahren zur Herstellung von 2-Chlor-5-alkylaminomethyl-pyridinen**
Process for the preparation of 2-chloro-5-alkylamino-methylpyridines
Procédé pour la préparation de 2-chloro-5-alkylamino-méthyl-pyridines

(30) Priorität: 19.02.1992 DE 4204919
(43) Veröffentlichungstag der Anmeldung: 25.08.1993
(73) Patentinhaber: BAYER AG, 51368 Leverkusen (DE)
(72) Erfinder: Diehr, Hans-Joachim, Dr., W-5600 Wuppertal 1 (DE)

(56) Entgegenhaltungen:
- EP-A- 0 302 389
- EP-A- 0 379 928
- EP-A- 0 425 030

## Beschreibung

Die Erfindung betrifft ein neues Verfahren zur Herstellung von 2-Chlor-5-alkylaminomethyl-pyridinen.

Es ist bekannt, daß man 2-Chlor-5-alkylaminomethyl-pyridine erhält, wenn man 2-Chlor-pyridin-5-carboxaldehyd zunächst mit Alkylaminen umsetzt und die dabei erhaltenen Alkyliminoverbindungen in einer zweiten Stufe mit Hydrierungsmitteln, wie z.B. Natriumboranat, behandelt (vgl. EP-A 302389).

Weiter ist bekannt, daß 2-Chlor-5-methylaminomethyl-pyridin durch Umsetzung von 2-Chlor-5-chlormethyl-pyridin mit Methylamin erhalten werden kann (vgl. EP-A 366085, EP-A 376279, GB-A 2228003, EP-A 425030).

Bei den beiden bekannten Synthesemethoden sind jedoch die erzielbaren Ausbeuten und die Qualitäten der Produkte nicht immer zufriedenstellend.

Es wurde nun gefunden, daß man 2-Chlor-5-alkylaminomethyl-pyridine der allgemeinen Formel (I) 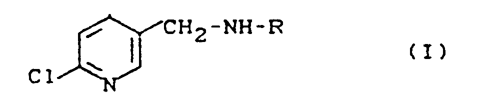
in welcher
- R: für Alkyl steht,
in sehr guten Ausbeuten und in hoher Reinheit erhält, wenn man
2-Chlor-5-aminomethyl-pyridin der Formel (II) 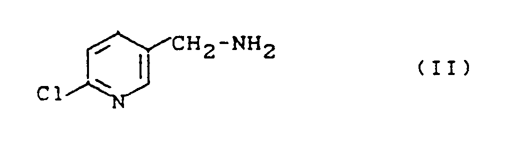
mit Ameisensäure, gegebenenfalls in Gegenwart eines Verdünnungsmittels, bei Temperaturen zwischen 20°C und 200°C umsetzt,
das hierbei gebildete 2-Chlor-5-formylaminomethyl-pyridin der Formel (III) 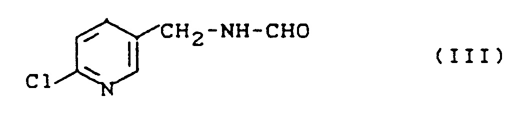
ohne Zwischenisolierung (in situ) mit Alkylierungsmitteln der allgemeinen Formel (IV)

R-X (IV)

in welcher
- X: für Halogen oder die Gruppierung -O-SO₂-O-R steht und
- R: die oben angegebene Bedeutung hat,
gegebenenfalls in Gegenwart eines Säureakzeptors, gegebenenfalls in Gegenwart eines Katalysators und gegebenenfalls in Gegenwart eines Verdünnungsmittels, bei Temperaturen zwischen 0°C und 100°C umsetzt
und schließlich die so gebildeten 2-Chlor-5-(N-alkyl-N-formyl-aminomethyl)-pyridine der allgemeinen Formel (V) 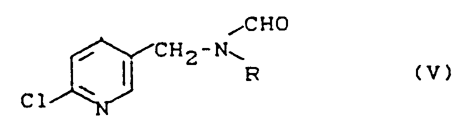
in welcher
- R: die oben angegebene Bedeutung hat,
gegebenenfalls nach Abdestillieren flüchtiger Komponenten, mit wäßriger Alkalilauge bei Temperaturen von 20°C bis Rückflußtemperatur umsetzt.

Überraschenderweise können nach dem erfindungsgemäßen Verfahren 2-Chlor-5-alkylaminomethyl-pyridine der Formel (I) in sehr guten Ausbeuten und in hoher Reinheit erhalten werden, obwohl dabei drei Reaktionsschritte durchlaufen werden, wobei keine Zwischenisolierung und damit auch keine Reinigung von Zwischenprodukten erfolgt.

Der Reaktionsablauf beim erfindungsgemäßen Verfahren kann beispielsweise durch das folgende Formelschema skizziert werden:

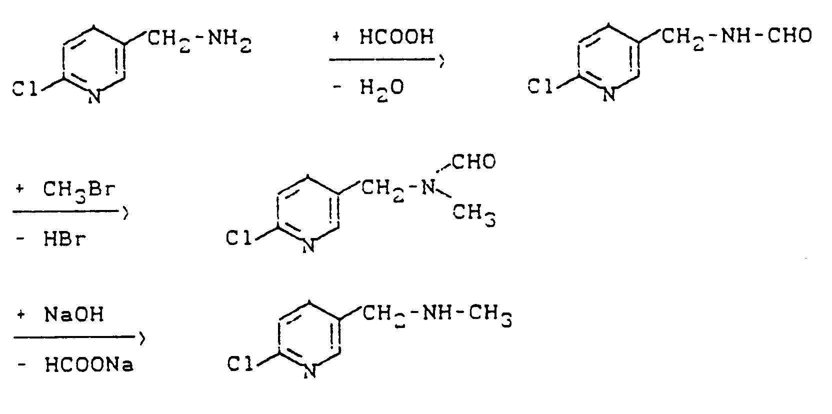

Das erfindungsgemäße Verfahren betrifft vorzugsweise die Herstellung von Verbindungen der Formel (I), in welcher
- R: für geradkettiges oder verzweigtes Alkyl mit 1 bis 6 Kohlenstoffatomen steht.

Inbesondere werden nach dem erfindungsgemäßen Verfahren Verbindungen der Formel (I) hergestellt, in welcher
- R: für Methyl, Ethyl, Propyl, Isopropyl, Butyl, Isobutyl oder sec-Butyl steht.

Das als Ausgangsverbindung zu verwendende 2-Chlor-5-aminomethyl-pyridin der Formel (II) ist bereits bekannt (vgl. EP-A 391205).

Das beim ersten Reaktionsschritt gebildete 2-Chlor-5-formylaminomethyl-pyridin der Formel (III) ist noch nicht aus der Literatur bekannt und als neue Verbindung Gegenstand der vorliegenden Patentanmeldung.

Die beim zweiten Reaktionsschritt als Ausgangsstoffe zu verwendenden Alkylierungsmittel sind durch die Formel (IV) allgemein definiert. In Formel (IV) stehen vorzugsweise
- X: für Chlor, Brom oder Iod, oder für die Gruppierung -O-SO₂-O-R, und
- R: für geradkettiges oder verzweigtes Alkyl mit 1 bis 6 Kohlenstoffatomen.

Insbesondere stehen in Formel (IV)
- X: für Brom oder Iod, oder für die Gruppierung -O-SO₂-O-R, und
- R: für Methyl, Ethyl, Propyl, Isopropyl, Butyl, Isobutyl oder sec-Butyl.

Die Ausgangsstoffe der Formel (IV) sind bekannte organische Synthesechemikalien.

Die beim zweiten Reaktionsschritt gebildeten 2-Chlor-5-(N-alkyl-N-formyl-aminomethyl)-pyridine der Formel (V) sind noch nicht aus der Literatur bekannt und sind als neue Verbindungen Gegenstand der vorliegenden Patentanmeldung.

In Formel (V) steht R vorzugsweise für geradkettiges oder verzweigtes Alkyl mit 1 bis 6 Kohlenstoffatomen, insbesondere für Methyl, Ethyl, Propyl, Isopropyl, Butyl, Isobutyl oder sec-Butyl.

Die ersten Schritte des erfindungsgemäßen Verfahrens werden Vorzugsweise in Gegenwart eines Verdünnungsmittels durchgeführt. Es kommen hierbei praktisch alle inerten organischen Lösungsmittel in Betracht. Hierzu gehören vorzugsweise aliphatische und aromatische, gegebenenfalls halogenierte Kohlenwasserstoffe wie Hexan, Heptan, Octan, Cyclohexan, Methylcyclohexan, Benzol, Toluol, Xylol, Chloroform, Tetrachlormethan, Chlorbenzol und o-Dichlorbenzol, sowie Ether wie Methyl-tert-butylether und Methyl-tert-pentylether. Toluol wird als Verdünnungsmittel ganz besonders bevorzugt.

Die Reaktionstemperaturen können in der ersten Phase des erfindungsgemäßen Verfahrens - zur Bildung des 2-Chlor-5-formylaminomethyl-pyridins der Formel (III) - in einem größeren Bereich variiert werden. Im allgemeinen arbeitet man bei Temperaturen zwischen 20°C und 200°C, vorzugsweise zwischen 50°C und 150°C.

Das erfindungsgemäße Verfahren wird im allgemeinen unter Normaldruck durchgeführt. Es ist jedoch auch möglich, unter erhöhtem oder vermindertem Druck zwischen 0,1 bar und 10 bar zu arbeiten.

Zur Durchführung der ersten Phase des erfindungsgemäßen Verfahrens setzt man auf 1 Mol 2-Chlor-5-aminomethyl-pyridin der Formel (II) im allgemeinen zwischen 1 und 2 Mol Ameisensäure, vorzugsweise zwischen 1,1 und 1,5 Mol Ameisensäure ein.

Das 2-Chlor-5-aminomethyl-pyridin, die Ameisensäure und das Verdünnungsmittel werden im allgemeinen bei Raumtemperatur vermischt und die Mischung wird dann, vorzugsweise unter Auskreisen des gebildeten Wassers am Wasserabscheider, zum Sieden erhitzt, bis die Umsetzung abgeschlossen ist. Die Reaktionslösung wird nach Abkühlen auf Raumtemperatur unmittelbar zur Durchführung der zweiten Phase des erfindungsgemäßen Verfahrens eingesetzt.

Die zweite Phase des erfindungsgemäßen Verfahrens - Bildung von 2-Chlor-5-(N-alkyl-N-formyl-aminomethyl)-pyridinen der Formel (V) - wird vorzugsweise in Gegenwart eines Säureakzeptors durchgeführt.

Als Säureakzeptoren können hierbei alle üblicherweise für derartige Umsetzungen verwendbaren Säurebindemittel eingesetzt werden. Vorzugsweise in Frage kommen Alkalimetall- und Erdalkalimetall-hydroxide, wie Lithium-, Natrium-, Kalium- und Calcium-hydroxid, Alkalimetall- und Erdalkalimetall-carbonate und -hydrogencarbonate, wie Natrium- und Kalium-carbonat oder -hydrogencarbonat sowie Calciumcarbonat, Alkalimetallacetate, wie Natrium- und Kalium-acetat, Alkalimetallalkoholate, wie Natrium- und Kalium-methylat, -ethylat, -propylat, -isopropylat, -butylat, -isobutylat und tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Alkalimetallhydroxide, insbesondere Natriumhydroxid und Kaliumhydroxid, vorzugsweise als 20%ige bis 50%ige wäßrige Lösung, werden als Säureakzeptoren besonders bevorzugt.

Die zweite Phase des erfindungsgemäßen Verfahrens (Bildung von Verbindungen der Formel (V)) wird gegebenenfalls in Gegenwart eines Katalysators durchgeführt. Als Katalysatoren sind hierbei insbesondere die sogenannten Phasentransfer-Katalysatoren, wie z.B. Triethyl-benzylammoniumchlorid (TEBA), Trimethyl-benzylammoniumchlorid moniumchlorid, Trimethyl-hexadecylammoniumchlorid, Trioctyl-methylammoniumchlorid, Tetrabutylammoniumbromid und Tetrabutyl-ammonium-hydrogensulfat geeignet.

Die Reaktionstemperaturen können in der zweiten Phase des erfindungsgemäßen Verfahrens in einem größeren Bereich variiert werden. Im allgemeinen arbeitet man bei Temperaturen zwischen 0°C und 100°C, vorzugsweise zwischen 10°C und 80°C.

Zur Durchführung der zweiten Phase des erfindungsgemäßen Verfahrens setzt man, bezogen auf 1 Mol des anfangs eingesetzten 2-Chlor-5-aminomethyl-pyridins der Formel (II), im allgemeinen zwischen 1 und 2 Mol, vorzugsweise zwischen 1,1 und 1,5 Mol, Alkylierungsmittel der Formel (IV) ein.

Die aus der ersten Verfahrensphase resultierende Reaktionslösung wird mit dem Katalysator und dem Alkylierungsmittel der Formel (IV) versetzt, wobei gegebenenfalls weiteres Verdünnungsmittel zugeführt wird; dann wird der Säureakzeptor langsam zudosiert und das Reaktionsgemisch bis zum Ende der Umsetzung gerührt; anschließend wird eingeengt. Der verbleibende Rückstand wird unmittelbar zur Durchführung des letzten Reaktionsschrittes eingesetzt.

Die dritte Phase des erfindungsgemäßen Verfahrens - Herstellung der 2-Chlor-5-alkylaminomethyl-pyridine der Formel (I) - wird unter Verwendung einer wäßrigen Alkalilauge durchgeführt. Vorzugsweise werden 10%ige bis 50%ige wäßrige Lösungen von Alkalimetallhydroxiden, insbesondere von Natriumhydroxid oder Kaliumhydroxid, eingesetzt.

Die Reaktionstemperaturen können in der dritten Phase des erfindungsgemäßen Verfahrens in einem größeren Bereich variiert werden. Im allgemeinen arbeitet man bei Temperaturen von 20°C bis Rückflußtemperatur, vorzugsweise zwischen 50°C und Rückflußtemperatur.

Zur Durchführung der dritten Phase des erfindungsgemäßen Verfahrens setzt man, bezogen auf 1 Mol des anfangs eingesetzten 2-Chlor-5-aminomethyl-pyridins der Formel (II), im allgemeinen zwischen 1 und 5 Mol, vorzugsweise zwischen 1,5 und 2,5 Mol Alkalimetallhydroxid ein.

Der aus der zweiten Verfahrensphase resultierende Rückstand, welcher im wesentlichen das Zwischenprodukt der Formel (V) enthält, wird mit der wäßrigen Alkalilauge versetzt und das Gemisch wird dann bei der erforderlichen Reaktionstemperatur bis zum Ende der Umsetzung gerührt.

Die Aufarbeitung kann auf übliche Weise durchgeführt werden. Beispielsweise wird nach dem Abkühlen mit einem mit Wasser praktisch nicht mischbaren organischen Lösungsmittel, wie z.B. Toluol, extrahiert und von der Extraktionslösung gegebenenfalls nach Trocknen, das Lösungsmittel unter vermindertem Druck sorgfältig abdestilliert. Der verbleibende Rückstand, welcher im wesentlichen das Produkt der Formel (I) enthält, kann auf übliche Weise weiter gereinigt oder direkt für weitere Umsetzungen verwendet werden.

Die nach dem erfindungsgemäßen Verfahren herzustellenden 2-Chlor-5-alkylaminomethyl-pyridine der Formel (I) können als Zwischenprodukte für Insektizide verwendet werden (vgl. EP-A 302389, EP-A 366085, EP-A 376279).

### Herstellungsbeispiele:

### Beispiel 1

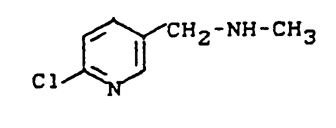

Eine Mischung aus 21,5 g (0,15 Mol) 2-Chlor-5-aminomethyl-pyridin, 8,3 g (0,18 Mol) Ameisensäure und 90 ml Toluol wird am Wasserabscheider 1 Stunde zum Sieden erhitzt. Anschließend wird auf Raumtemperatur (ca. 20°C) abgekühlt und es werden 0,5 g Triethyl-benzylammoniumchlorid und eine Lösung von 22,7 g (0,18 Mol) Dimethyl-sulfat in 70 ml Toluol dazu gegeben. Dann wird eine Lösung von 30 g (0,75 Mol) Natriumhydroxid in 65 ml Wasser langsam eindosiert und die Reaktionsmischung wird noch 30 Minuten bei 20°C gerührt; das Toluol wird dann abdestilliert. Zum Rückstand wird eine Lösung von 12 g (0,3 Mol) Natriumhydroxid in 48 ml Wasser gegeben und das Gemisch wird 2 Stunden unter Rückfluß erhitzt. Nach dem Erkalten wird mit 2x 100 ml Toluol extrahiert, die organischen Extrakte vereinigt, mit Natriumsulfat getrocknet und filtriert. Vom Filtrat wird das Lösungsmittel, zunächst im Wasserstrahlvakuum, dann im Ölpumpenvakuum sorgfältig abdestilliert.

Man erhält 22,5 g 2-Chlor-5-methylaminomethyl-pyridin als öligen Rückstand. Siedepunkt: 100-103°C bei 3 bis 4 bar.

Gehalt nach HPLC: 90%; damit errechnet sich eine Ausbeute von 92% der Theorie.

In analoger Weise ist die Verbindung 2-Chlor-5-ethylaminomethyl-pyridin vom Siedepunkt 89-91°C bei 0,1 bis 0,3 bar erhältlich.

## Patentansprüche

1. Verfahren zur Herstellung von 2-Chlor-5-alkylaminomethyl-pyridinen der allgemeinen Formel (I) 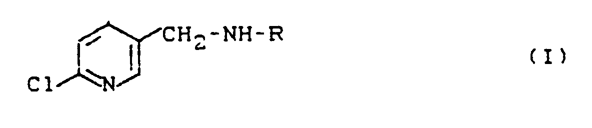 in welcher
R für Alkyl steht,
dadurch gekennzeicnet, daß man 2-Chlor-5-aminomethyl-pyridin der Formel (II) 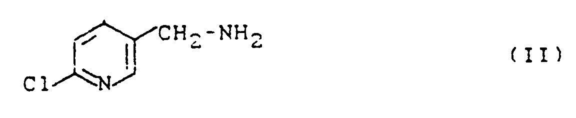 mit Ameisensäure, gegebenenfalls in Gegenwart eines Verdünnungsmittels, bei Temperaturen zwischen 20°C und 200°C umsetzt,
das hierbei gebildete 2-Chlor-5-formylaminomethyl-pyridin der Formel (III) 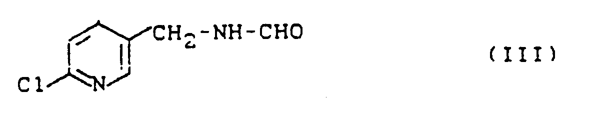 ohne Zwischenisolierung (in situ) mit Alkylierungsmitteln der allgemeinen Formel (IV)
R-X (IV)
in welcher
X für Halogen oder die Gruppierung -O-SO₂-O-R steht und
R die oben angegebene Bedeutung hat,
gegebenenfalls in Gegenwart eines Säureakzeptors, gegebenenfalls in Gegenwart eines Katalysators und gegebenenfalls in Gegenwart eines Verdünnungsmittel, bei Temperaturen zwischen 0°C und 100°C umsetzt und schließlich die so gebildeten 2-Chlor-5-(N-alkyl-N-formyl-aminomethyl)-pyridine der allgemeinen Formel (V) 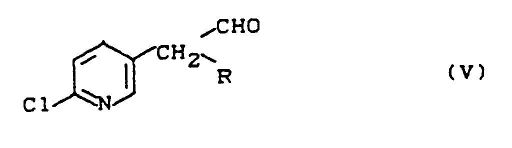 in welcher
R die oben angegebene Bedeutung hat,
gegebenenfalls nach Abdestillieren flüchtiger Komponenten, mit wäßriger Alkalilauge bei Temperaturen zwischen 20°C und Rückflußtemperatur umsetzt.

2. Verfahren gemäß Anspruch 1 zur Herstellung von Verbindungen der Formel (I), in welcher
R für geradkettiges oder verzweigtes Alkyl mit 1 bis 6 Kohlenstoffatomen steht.

3. Verfahren gemäß Anspruch 1 zur Herstellung von Verbindungen der Formel (I), in welcher
R für Methyl, Ethyl, Propyl, Isopropyl, Butyl, Isobutyl oder sec-Butyl steht.

4. Verfahren gemäß Ansprüchen 1 bis 3, dadurch gekennzeichnet, daß man pro Mol 2-Chlor-5-aminomethylpyridin der Formel (II) 1 bis 2 Mol Ameisensäure einsetzt.

5. Verfahren gemäß Ansprüchen 1 bis 3, dadurch gekennzeichnet, daß man die Bildung von Verbindungen der Formel (V) in Gegenwart von Phasentransfer-Katalysatoren durchführt.

6. Verfahren gemäß Ansprüchen 1 bis 4, dadurch gekennzeichnet, daß man bei der Bildung der Verbindungen der Formel (V) bezogen auf 1 Mol des anfangs eingesetzten 2-Chlor-5-aminomethyl-pyridins (II) zwischen 1 und 2 Mol Alkylierungsmittel der Formel (IV) einsetzt.

7. Verfahren gemäß Ansprüchen 1 bis 3, dadurch gekennzeichnet, daß man in der dritten Phase des Verfahrens bezogen auf 1 Mol des anfangs eingesetzten 2-Chlor-5-aminomethyl-pyridins (II) zwischen 1 und 5 Mol Alkalimetallhydroxid einsetzt.

8. Verfahren gemäß Anspruch 7, dadurch gekennzeichnet, daß man eine 10 bis 50%ige wäßrige Lösung von Alkalimetallhydroxiden einsetzt.

9. 2-Chlor-5-formylaminomethyl-pyridin der Formel (III)

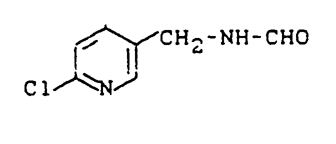

10. 2-Chlor-5-(N-alkyl-N-formyl-aminomethyl)-pyridine der Formel (IV) 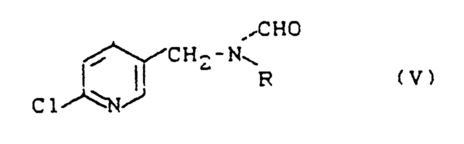 in welcher
R für Alkyl steht.

## Claims

1. Process for the preparation of 2-chloro-5-alkyl-aminomethyl-pyridines of the general formula (I) 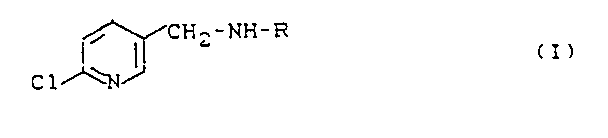 in which
R represents alkyl,
characterised in that 2-chloro-5-aminomethyl-pyridine, of the formula (II), 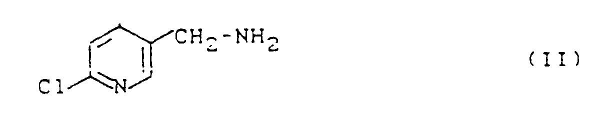 is reacted with formic acid at temperatures between 20°C and 200°C, if appropriate in the presence of a diluent,
the 2-chloro-5-formylaminomethyl-pyridine which has been formed in this process, of the formula (III), 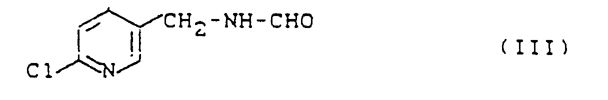 is reacted without intermediate isolation (in situ) with alkylating agents of the general formula (IV)
R-X (IV)
in which
X represents halogen or the group -O-SO₂-O-R and
R has the abovementioned meaning,
at temperatures between 0°C and 100°C, if appropriate in the presence of an acid acceptor, if appropriate in the presence of a catalyst and if appropriate in the presence of a diluent, and, finally, the 2-chloro-5-(N-alkyl-N-formyl-aminomethyl)-pyridines thus formed of the general formula (V) 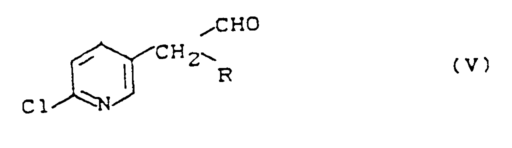 in which
R has the abovementioned meaning,
are reacted with aqueous alkali metal hydroxide solution at temperatures between 20°C and reflux temperature, if appropriate after volatile components have been distilled off.

2. Process according to Claim 1 for the preparation of compounds of the formula (I) in which
R represents straight-chain or branched alkyl having 1 to 6 carbon atoms.

3. Process according to Claim 1 for the preparation of compounds of the formula (I) in which
R represents methyl, ethyl, propyl, isopropyl, butyl, isobutyl or sec-butyl.

4. Process according to Claims 1 to 3, characterised in that 1 to 2 moles of formic acid are employed per mole of 2-chloro-5-aminomethylpyridine, of the formula (II).

5. Process according to Claims 1 to 3, characterised in that the formation of compounds of the formula (V) is carried out in the presence of phase transfer catalysts.

6. Process according to Claims 1 to 4, characterised in that, in the formation of the compounds of the formula (V), between 1 and 2 moles of alkylating agent of the formula (IV) are employed relative to 1 mole of the initially employed 2-chloro-5-aminomethyl-pyridine (II).

7. Process according to Claims 1 to 3, characterised in that, in the third phase of the process, between 1 and 5 moles of alkali metal hydroxide are employed relative to 1 mole of the initially employed 2-chloro-5-aminomethyl-pyridine (II).

8. Process according to Claim 7, characterised in that a 10 to 50% strength aqueous solution of alkali metal hydroxides is employed.

9. 2-Chloro-5-formylaminomethyl-pyridine, of the formula (III)

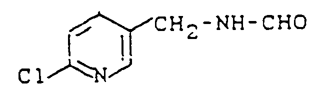

10. 2-Chloro-5-(N-alkyl-N-formyl-aminomethyl)-pyridines of the formula (IV) 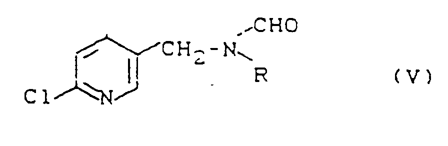 in which
R represents alkyl.

## Revendications

1. Procédé de préparation de 2-chloro-5-alkylaminométhyl-pyridines de la formule générale (I) 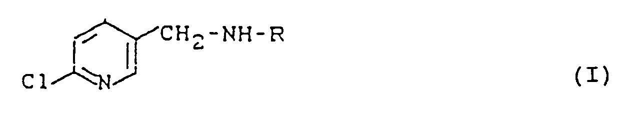 dans laquelle
R représente un alkyle,
caractérisé en ce que l'on fait réagir une 2-chloro-5-aminométhyl-pyridine de formule (II) 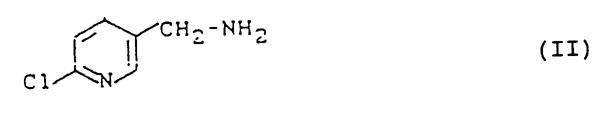 avec l'acide formique, éventuellement en présence d'un agent de dilution, à des températures situées entre 20°C et 200°C,
en ce que la 2-chloro-5-formylaminométhyl-pyridine de formule (III) ainsi formée 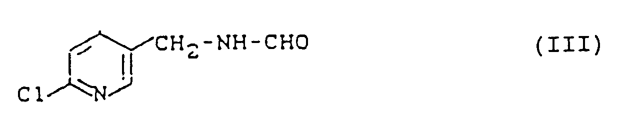 est mise à réagir sans isolation intermédiaire (in situ) avec des agents d'alkylation de formule générale (IV)
R-X (IV)
dans laquelle
X représente un halogène ou le groupe -O-SO₂-O-R, et
R a la même signification que celle donnée plus haut,
éventuellement en présence d'un accepteur d'acide, éventuellement en présence d'un catalyseur et éventuellement en présence d'un agent de dilution, à des températures situées entre 0°C et 100°C, et en ce qu'enfin la 2-chloro-5-(N-alkyl-N-formylaminaméthyl)-pyridine ainsi formée, de formule générale (V) 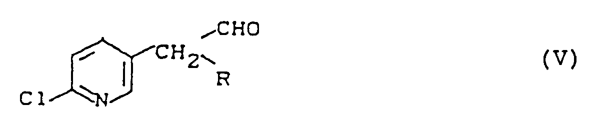 dans laquelle
R a la même signification que celle donnée plus haut,
est mise à réagir avec une solution aqueuse d'un alcali, à des températures situées entre 20°C et la température de reflux, éventuellement après extraction de composants volatils par distillation.

2. Procédé selon la revendication 1, pour la préparation de composés de formule (I), dans laquelle
R représente un alkyle à chaîne linéaire ou ramifiée comprenant de 1 à 6 atomes de carbone.

3. Procédé selon la revendication 1, pour la préparation de composés de formule (I), dans laquelle
R représente le méthyle, l'éthyle, le propyle, l'isopropyle, le butyle, l'isobutyle ou le sec-butyle.

4. Procédé selon les revendications 1 à 3, caractérisé en ce que par mole de 2-chloro-5-aminométhyl-pyridine de formule (II), on utilise 1 à 2 moles d'acide formique.

5. Procédé selon les revendications 1 à 3, caractérisé en ce que l'on réalise la formation de composés de formule (V) en présence de catalyseurs de transfert de phase.

6. Procédé selon les revendications 1 à 4, caractérisé en ce que lors de la formation des composés de formule (V), on utilise, pour 1 mole de la 2-chloro-5-aminométhyl-pyridine (II) utilisée au début, entre 1 et 2 moles d'agent d'alkylation de formule (IV).

7. Procédé selon les revendications 1 à 3, caractérisé en ce qu'au cours de la troisième phase du procédé, on utilise, pour 1 mole de la 2-chloro-5-aminométhyl-pyridine (II) utilisée au début, entre 1 et 5 moles d'hydroxyde de métal alcalin.

8. Procédé selon la revendication 7, caractérisé en ce que l'on utilise une solution aqueuse de 10 à 50% d'hydroxydes de métaux alcalins.

9. 2-chloro-5-formylaminométhyl-pyridine de formule (III)

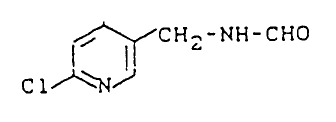

10. 2-chloro-5-(N-alkyl-N-formylaminométhyl)-pyridine de formule (V) 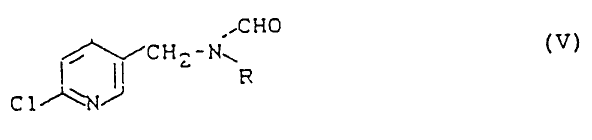 dans laquelle
R représente un alkyle.
